# EUROPEAN PATENT APPLICATION

(11) **EP 2 133 074 A1**
(43) Date of publication of application: **16.12.2009**
(21) Application number: 08290531.6
(22) Date of filing: 10.06.2008
(51) Int. Cl.: A61K 31/343, A61P 9/06

(54) **Use of dronedarone for the preparation of a medicament intended for the prevention of permanent atrial fibrillation**

(71) Applicant: Sanofi-Aventis, 75013 Paris (FR)
(72) Inventor: Gaudin, Christophe, 75013 Paris (FR); Hamdani, Nacéra, 75013 Paris (FR); Radzik, David, 75013 Paris (FR); Van Eickels, Martin, 65926 Frankfurt am Main (DE)
(74) Representative: Romanowski, Caroline

(57) **Abstract**

Use of dronedarone for the preparation of a medicament intended for the prevention of permanent atrial fibrillation.

## Description

The instant invention relates to the use of dronedarone for the preparation of a medicament intended for the prevention of permanent atrial fibrillation.

2-n-butyl-3-[4-(3-di-n-butylaminopropoxy)benzoyl]-5-méthylsulfonamido-benzofuranne or dronedarone and its pharmaceutically acceptable salts are described in European patent EP 0 471 609 B1.
Dronedarone is a multi-channel blocker that affects calcium, potassium and sodium channels and has anti-adrenergic properties.
Dronedarone is an anti-arrhythmic agent for the treatment of patients with a history of atrial fibrillation or atrial flutter.
Atrial fibrillation (AF) affects about 2.3 million people in North America and 4.5 million people in the European Union and is emerging as a growing public health concern because of the aging of the population
AF is a condition in which the upper chambers of the heart beat in an uncoordinated and disorganized fashion, resulting in a very irregular and fast rhythm (i.e., an irregularly, irregular heartbeat). When blood is not completely pumped out of the heart's chambers, it can pool and clot. If a blood clot forms in the atria, exits the heart and blocks an artery in the brain, a stroke results. Consequently, about 15 percent of strokes result from AF.
AF is increasingly frequent with advancing age and is often caused by age-related changes in the heart, physical or psychological stress, agents that stimulate the heart, such as caffeine, or as a result of cardiovascular disease. The number is expected to double in the next 20 years. Without appropriate management, AF can lead to serious complications, such as stroke and congestive heart failure.

It is know that atrial fibrillation itself can cause changes in the electrical parameters of the heart known as electrical remodelling and in the structure of the cardiac chambers known as structural remodelling which tend to decrease the chances of the patient to get back into normal sinus rhythm. This vicious circle whereby "atrial fibrillation begets atrial fibrillation" has been well documented since the 1990s (Wijffels MC, Kirchhof CJ, Dorland R, Allessie MA.Atrial fibrillation begets atrial fibrillation. A study in awake chronically instrumented goats.Circulation. 1995 Oct 1;92(7):1954-68.). It explains why when patients have been in atrial fibrillation for a long time they develop permanent atrial fibrillation with little or no chance to recover from this arrhythmia which becomes chronic.

The Inventors have now found that dronedarone reduces the likelihood of staying in permanent AF and thus prevents patients from permanent atrial fibrillation/flutter.

The subject of the instant invention is the use of dronedarone or one of its pharmaceutically acceptable salts for the preparation of a medicament intended to the prevention of permanent atrial fibrillation/flutter in patients with a history of atrial fibrillation or atrial flutter.

More precisely, the invention relates to the use of dronedarone or one of its pharmaceutically acceptable salts for the preparation of a medicament intended to the prevention of about 33 % of cardiovascular hospitalization or death of patients with permanent atrial fibrillation.

The percentage above corresponds to an average.

Among the pharmaceutically acceptable salts of dronedarone, mention may be made of the hydrochloride.

The expression « with a history of atrial fibrillation or atrial flutter » means a patient who has a history of atrial fibrillation (AF) or atrial flutter (AFL) and who can be either in sinus rhythm or in atrial fibrillation or atrial flutter at the time of dronedarone administration.

In the instant invention, "atrial fibrillation" means atrial fibrillation and/or atrial flutter.

Among patients with a history of atrial fibrillation or atrial flutter, mention may be made of patients who further have at least one of the following risk factors :
- hypertension,
- diabete,
- prior cerebrovascular accident or systemic embolism,
- left atrium diameter greater that or equal to 50 mm by echocardiography,
- left ventricular ejection fraction less than 40% by 2D-echocardiography.

Among patients with a history of atrial fibrillation or atrial flutter, mention may also be made of patients having additional risk factors corresponding to at least one of the following diseases:
- hypertension,
- structural heart disease,
- tachycardia,
- coronary heart disease,
- non-rheumatic valvular heart disease,
- ischemic dilated cardiomyopathy,
- a history of ablation for AF/AFL for example catheter ablation or surgical ablation,
- supra-ventricular tachycardia other than AF/AFL,
- history of cardiac valve surgery,
- non-ischemic dilated cardiomyopathy,
- hypertrophic cardiomyopathy,
- rheumatic valvular heart disease,
- sustained ventricular tachycardia,
- congenital heart disease,
- a history of ablation for other reason than AF/AFL for example catheter ablation,
- ventricular fibrillation,
and/or at least a cardiac device chosen among:
- a pacemaker,
- an implanted cardioverter defibrillator.

Cardiovascular hospitalizations means hospitalization having for main causes one of the following diseases : (Hohnloser and al., Journal of cardiovascular electrophysiology, janv 2008, vol. 19, n°1, pages 69-73) :
- atherosclerosis related,
- myocardial infarction or unstable angina,
- stable angina pectoris or atypical chest pain,
- syncope,
- TIA or stroke (except intracranial haemorrhage),
- Atrial fibrillation and other supraventricular rhythm disorders,
- Non fatal cardiac arrest,
- Ventricular arrhythmia,
- Cardiovascular surgery except cardiac transplantation,
- cardiac transplantation,
- implantation of a pacemaker, of a implantable cardioverter defibrillator (« ICD ») or any other cardiac device,
- transcutaneous coronary, cerebrovascular or peripheral procedure,
- blood pressure related (hypotension, hypertension, except syncope),
- cardiovascular infection,
- major bleeding (requiring two or more units of blood or any intracranial haemorrhage),
- pulmonary embolism or deep vein thrombosis,
- worsening CHF including pulmonary edema or dyspnea of cardiac origin.

"Death" means death from any cause, cardiovascular or non cardiovascular.

Another object of the invention is a pharmaceutical composition which comprises, as active principle, a compound of formula (I) according to the present invention. This pharmaceutical composition comprises an effective dose of at least one compound of formula (I) according to the invention, or an addition salt thereof with a pharmaceutically acceptable salt, or a hydrate or solvate thereof, and at least one pharmaceutically acceptable excipient. Said excipients are chosen according to the pharmaceutical form and the administration route desired, among usual excipients known to one of skill in the art.

In the pharmaceutical compositions according to the invention for the oral, sublingual, sub-cutaneous, intramuscular, intra-venous, topical, local, intratracheal, intranasal, transdermal or rectal administration, the active principle of formula (I) above or its salt, solvate or hydrate, can be administered as a unitary dosage form, in blend with usual pharmaceutical excipients, to animals and human beings for the prevention or for the treatment of pathological states mentioned above. The appropriate unitary dosage forms comprise the oral forms, such as tablets, hard or soft gelatin capsules, powders, granules and oral solutions or suspensions, the sublingual, buccal, intratracheal, intraocular, intranasal forms, the forms adapted to inhalation, topical, transdermal, sub-cutaneous, intramuscular or intra-venous delivery, the rectal forms and the implants. For the topical application, the compounds of the invention may be used as creams, gels, ointments or lotions.

For its use in therapeutics, dronedarone and its pharmaceutically acceptable salts are incorporated in pharmaceuticals compositions.
These pharmaceutical compositions comprise an effective dose of at least dronedarone or one of its pharmaceutically acceptable salts and at least one pharmaceutically acceptable excipient.
Said excipients are chosen according to the pharmaceutical form and the administration route desired, among usual excipients known of one of skill in the art.

In the pharmaceutical compositions for the oral, sublingual, sub-cutaneous, intramuscular, intra-venous, topical, local, intratracheal, intranasal, transdermal or rectal administration, dronedarone or one of its pharmaceutically acceptable salts, can be administered as a unitary dosage form, in blend with usual pharmaceutical excipients, to animals and human in diseases above mentioned.

The appropriate unitary dosage forms comprise the oral forms, such as tablets, hard or soft gelatin capsules, powders, granules and oral solutions or suspensions, the sublingual, buccal, intratracheal, intraocular, intranasal forms, by inhalation, the topical, transdermal, sub-cutaneous, intramuscular or intra-venous forms, the rectal forms and the implants. For the topical application, the compounds of the invention may be used as creams, gels, ointments or lotions.

As an example, a unitary dosage form for dronedarone or one of its pharmaceutically acceptable salts, in the form of a tablet, can comprise the following ingredients:

| Ingredients | mg |
|---|---|
| dronedarone hydrochloride (corresponding to 400 mg of base) | 426 |
| Methylhydroxypropylcellulose | 21,1 |
| Lactose monohydrate | 46,55 |
| Modified corn starch | 45,5 |
| Polyvinylpyrrolidone | 65 |
| Poloxamer 407 | 40 |
| Anhydrous colloidal silica | 2,6 |
| magnesium stearate | 3,25 |
| | 650 |

| Ingredients | mg |
|---|---|
| dronedarone hydrochloride (corresponding to 400 mg of base) | 426 |
| microcristalline cellulose | 65 |
| Anhydrous colloidal silica | 2,6 |
| anhydrous lactose | 42,65 |
| Polyvinylpyrrolidone | 13 |
| Poloxamer 407 | 40 |
| Macrogol 6000 | 57,5 |
| magnesium stearate | 3,25 |
| | 650 |

| Ingredients | mg |
|---|---|
| dronedarone hydrochloride (corresponding to 400 mg of base) | 426 |
| microcristalline cellulose | 26 |
| corn starch | 45,5 |
| Polyvinylpyrrolidone | 65 |
| Poloxamer 407 | 40 |
| Anhydrous colloidal silica | 3,25 |
| magnesium stearate | 3,25 |
| Lactose monohydrate | 41,65 |
| | 650 |

| Ingredients | mg |
|---|---|
| dronedarone hydrochloride (corresponding to 400 mg of base) | 213 |
| microcrystalline cellulose | 13 |
| corn starch | 22,75 |
| Polyvinylpyrrolidone | 32,5 |
| Poloxamer 407 | 20 |
| Anhydrous colloidal silica | 1,3 |
| magnesium stearate | 1,625 |
| Lactose monohydrate | 20,825 |
| | 650 |

For oral administration, dronedarone daily dose may reach 800 mg.

In specific cases, higher or lower dosages may be appropriate; these dosages are comprised within the scope of the present invention. According to usual practice, the dosage suitable to each patient is determined by the physician according to the administration route, the weight, the disease, the body surface, the cardiac output and response of the patient.

The instant invention also relates to a method of treatment of the above mentioned disease which comprises the administration to a patient of an effective dose of at least dronedarone or one of its pharmaceutically acceptable salts.

The invention is illustrated with the above data with reference to the following figure:
Figure 1 represents Prentice cumulative incidence curves from randomization to first cardiovascular hospitalization or death from any cause according to the on-treatment analysis of 30 months - all randomized and treated patients with permanent AF/AFL on treatment.

Efficacy of dronedarone and its pharmaceutically acceptable salts versus placebo for the prevention of permanent atrial fibrillation was provided via dronedarone hydrochloride during a prospective, multinational, double-blind, randomized, multi-center, placebo-controlled, parallel group trial.

### I. Selection of patients

Patients must have a history of atrial fibrillation or atrial flutter and/or may be in normal sinus rhythm or in atrial fibrillation or flutter at the time of recruitment.

Recruitment of patients was conducted taking into account the following inclusion criteria:

### Inclusion criteria :

1) One of the following risk factors must be present :
   - Age equal or greater that 70 years, or above 75 years, associated or not with at least one of the following risk factors :
      o hypertension (taking antihypertensive drugs of at least two different classes),
      o diabetes,
      o prior cerebrovascular accident (stroke or transient ischemic attack) or systemic embolism,
      o left atrium diameter greater that or equal to 50 mm by echocardiography,
      o left ventricular ejection fraction less than 40% by 2D-echocardiography,
2) availability of one electrocardiogram within the last six months, showing that the patients was or is in atrial fibrillation/flutter,
3) availability of one electrocardiogram within the last six months, showing that the patients was or is in sinus rhythm.

### II. Duration and treatment

Study drug treatment units (placebo or dronedarone hydrochloride corresponding to 400 mg of base) were such that each patient took one tablet in the morning during or shortly after breakfast and one tablet in the evening during or shortly after dinner.

The treatment duration depended on the time of recruitment of each patient in the trial and could be comprised from 12 months to 30 months.

### III. Results

From the 4628 patients included in the trial, 2301 were part of the group treated with dronedarone hydrochloride.

"Patients with permanent atrial fibrillation or flutter" means patients with a history of atrial fibrillation or atrial flutter, who were in atrial fibrillation or flutter at the time of recruitment but who had not a previous history of permanent atrial fibrillation or flutter and who stayed in permanent atrial fibrillation or flutter during all the trial.
Results relating to the prevention of permanent atrial fibrillation/flutter

The number of patients with permanent atrial fibrillation/flutter was compared using Fischer's exact test.
294 patients had permanent atrial fibrillation/flutter in the group treated with placebo versus 178 patients in the group treated with dronedarone hydrochloride (p<0.001).
It indicates a decrease of the likelihood of developing permanent AF. Consequently, dronedarone prevents the risk of permanent atrial fibrillation/flutter.

## Claims

1. Use of dronedarone for the preparation of a medicament intended for the prevention of permanent atrial fibrillation/flutter.

2. Use according to claim 1 for the preparation of a medicament intended for the prevention of about 33 % of cardiovascular hospitalization or death for patients with permanent atrial fibrillation/flutter.

3. Use according to claim 1 or 2, **characterized in that** the patients have a history of atrial fibrillation or atrial flutter.

4. Use according to 1, 2 or 3, **characterized in that** the patients have at least one of the following risk factors :
- hypertension,
- diabete,
- prior cerebrovascular accident or systemic embolism,
- left atrium diameter greater that or equal to 50 mm by echocardiography,
- left ventricular ejection fraction less than 40% by 2D-echocardiography.

5. Use according to 1, 2, 3 or 4, **characterized in that** the patients have additional risk factors corresponding to at least one of the following diseases:
- hypertension,
- structural heart disease,
- tachycardia,
- coronary heart disease,
- non-rheumatic valvular heart disease,
- ischemic dilated cardiomyopathy,
- ablation for AF/AFL,
- supra-ventricular tachycardia other than AF/AFL,
- history of cardiac valve surgery,
- non-ischemic dilated cardiomyopathy,
- hypertrophic cardiomyopathy,
- rheumatic valvular heart disease,
- sustained ventricular tachycardia,
- congenital heart disease,
- ablation for other reason than AF/AFL,
- ventricular fibrillation,
and/or at least a cardiac device chosen among:
- a pacemaker,
- an implanted cardioverter defibrillator.

6. Use according to one of the preceding claims, **characterized in that,** for oral administration, dronedarone daily dose may reach 800 mg.
